# EUROPEAN PATENT APPLICATION

(11) **EP 2 423 178 A1**
(43) Date of publication of application: **29.02.2012**
(21) Application number: 10171078.8
(22) Date of filing: 28.07.2010
(51) Int. Cl.: C07C 69/65, C07C 271/22, C07D 487/04

(54) **Process for the production of sitagliptin**

(71) Applicant: Chemo Ibérica, S.A., 08028 Barcelona (ES)
(72) Inventor: Rasparini, Marcello, 27010 Cura Carpignano (IT); Colombo, Lino, 27100 Pavia (IT); D'Arienzo Giuseppe, 82100 Benevento (IT); Tufaro, Roberto, 28060 Sozzago (IT); Venegoni, Serena, 21052 Busto Arsizio (IT)
(74) Representative: Palladino, Saverio Massimo

(57) **Abstract**

The present invention is directed to a process for the preparation of Sitagliptin, having formula (I)

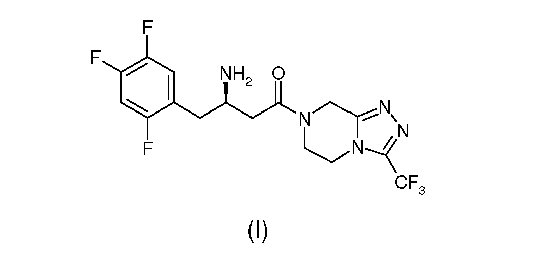

## Description

### Field of the invention

The present invention relates to a process for the production of Sitagliptin.

### Background of the invention

Sitagliptin is the compound having IUPAC name 7-[(3*R*)-3-amino-1-oxo-4-(2,4,5-trifluorophenyl)butyl]-5,6,7,8-tetrahydro-3-(trifluoromethyl)-1,2,4-triazolo[4,3-a]pyrazine, and the following formula (I):

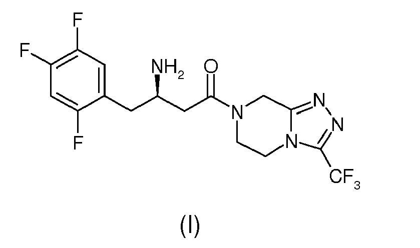

This compound belongs to a class of beta-amino tetrahydrotriazolo-[4,3-a]pyrazines, which are potent second generation inhibitors of dipeptidyl-peptidase IV (DP-IV), and therefore useful for the treatment of Type-2 diabetes. It is marketed as monophosphate salt and monohydrate adduct under the trade name Januvia^{®} (Trademark of Merck & Co.).

Sitagliptin, and a possible way for its preparation, are disclosed in international patent application WO 03/004498 A1. Once obtained, the compound can be purified according to methods standard in the field, obtaining Sitagliptin of purity degree suitable for pharmaceutical applications.

An improved synthetic route for the preparation of the compound is described in the paper "Highly Efficient Asymmetric Synthesis of Sitagliptin", K. B. Hansen et al.: Journal of the American Chemical Society (2009), 131 (25), 8798-8804, and in international patent application WO 2004/085378 A1. The characteristic step of the process of these documents is the enantioselective hydrogenation of a prochiral enamine in the presence of a transition metal precursor complexed to a chiral ferrocenyl diphosphine ligand. This synthetic pathway is likely the most convenient one among those presently known.

### Summary of the invention

It is an object of the present invention to provide an alternative process for the production of Sitagliptin.

The present invention provides a new process for the production of Sitagliptin comprising the steps of:
1) reacting itaconic acid (II) first with methyl alcohol and then with benzyl bromide, obtaining 1-benzyl-4-methyl-2-methylenesuccinate (III): 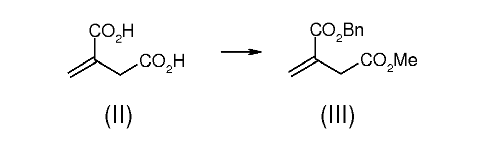 in which Bn indicates the benzyl radical;
2) reacting compound (III) with 1-bromo-2,4,5-trifluorobenzene to obtain (*E*)-1-benzyl-4-methyl-2-(2,4,5-trifluorobenzylidene)succinate, compound (IV): 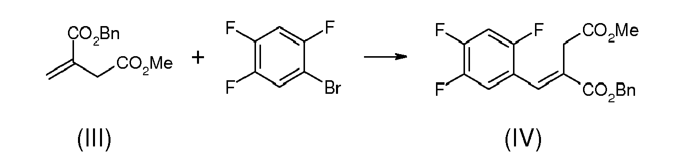
3) debenzylating compound (IV) to obtain (*E*)-4-methoxy-4-oxo-2-(2,4,5-trifluorobenzylidene)butanoic acid, compound (V): 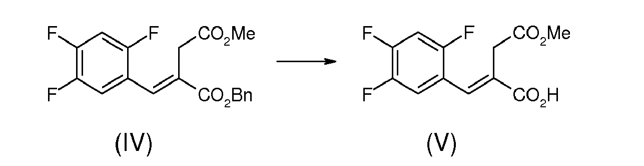
4) transforming compound (V) into (*R*)-4-methoxy-4-oxo-2-(2,4,5-trifluorobenzyl)butanoic acid, compound (VI): 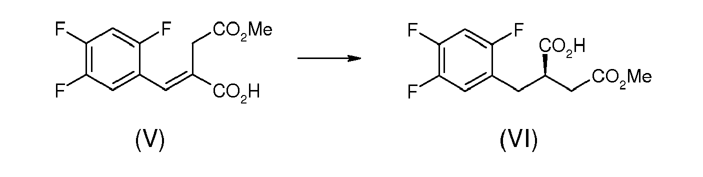 through 4a) catalytic hydrogenation followed by optical resolution, or 4b) asymmetric hydrogenation;
5) transforming compound (VI) through the Curtius or an analogous reaction into a carbamate of general formula (VII): 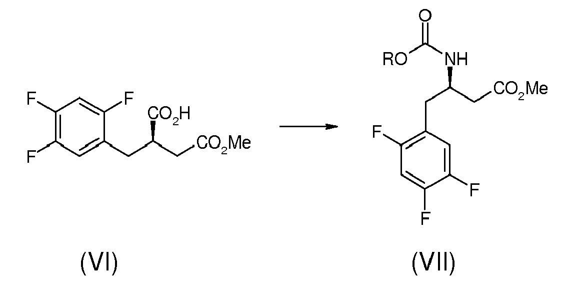
6) hydrolizing the methyl-ester group of carbamate (VII) to obtain the corresponding acid (VIII): 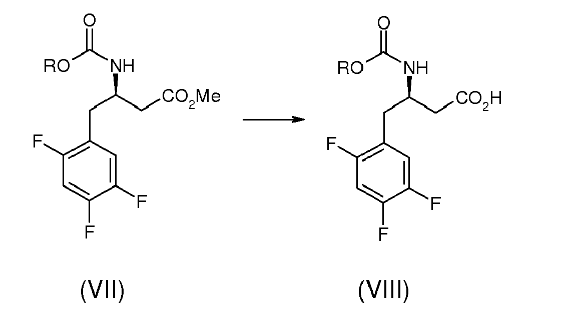
7) reacting acid (VIII) with 3-(trifluoromethyl)-5,6,7,8-tetrahydro-[1,2,4]triazolo[4,3-a]pyrazine, compound (IX), to obtain amide (X): 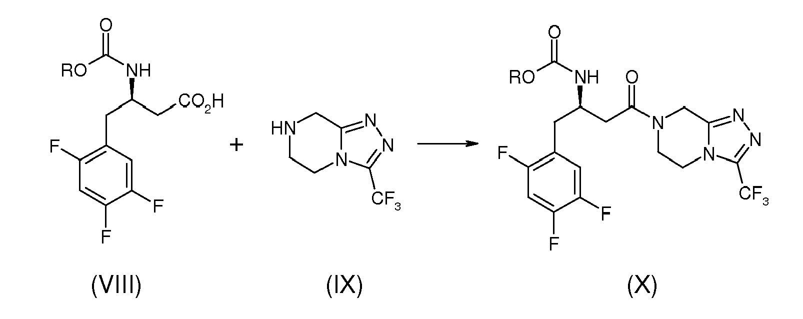
8) transforming the carbamate group of compound (X) into the corresponding amino group, thus obtaining Sitagliptin (I):

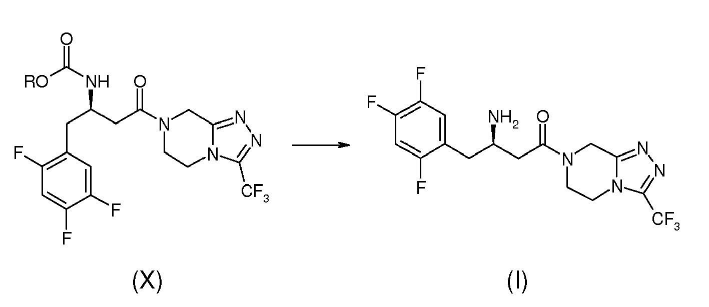

Further objects of the invention are the intermediate compounds (IV), (V), (VI), and compounds (VII), (VIII) and (X) in which R = benzyl, and the salts thereof in case the compounds are acids, produced in the process above.

### Detailed description of the invention

The first step of the process of the invention is the transformation of itaconic acid (II) into mono-methyl ester (III). Itaconic acid (IUPAC name 2-methylidenebutanedioic acid, also called methylenesuccinic acid) is commercially available from essentially any reseller of chemicals.

The production of the double ester (III) takes place with two subsequent substeps. In the first sub-step a first esterification with methanol is carried out in conditions according to the Fischer reaction (acid catalysis). This first esterification is preferably carried out in absence of a co-solvent, the reaction medium being methanol used in excess; the acid used as catalyst can be, e.g., sulfuric acid or p-toluensulfonic acid, or thionyl chloride (SOCl₂). Under these conditions, the methylation reaction occurs essentially only at the carboxylic group farther from the methylene group of itaconic acid; small amounts of double methyl ester are formed, that can be removed by extraction.

The second esterification is carried out with benzylbromide in the presence of an inorganic base in an organic polar aprotic solvent, resulting in compound (III). The reaction is carried out at temperatures comprised between 25 and 35°C, and the yield is greater than 80%.

In the second step of the process of the invention, compound (III) is reacted with 1-bromo-2,4,5-trifluorobenzene, in a cross-coupling reaction. The reaction is catalyzed by a palladium-based catalyst, and is carried out in the presence of a base, optionally of an alkaline bromide or a tetraalkylammonium bromide, and in an organic polar aprotic solvent, at a temperature comprised between about 50 and 150°C, and preferably between about 90 and 110°C.

The catalyst may be a palladium salt, such as palladium chloride or acetate, optionally in the presence of phosphine bidentate ligands such as dppf (diphenylphosphine ferrocene); it is also possible to use metallic palladium on carbon, that however gives rise to reduced reaction yield. The preferred catalyst is palladium acetate with no ligands.

The base can be inorganic, such as Na₂CO₃ or K₂CO₃, or a tertiary amine such as triethylamine, ethyl di-isopropyl amine, or, preferably, dicyclohexyl methyl amine; the amount of base can vary between about 1 and 2, preferably about 1.5, equivalents.

When bromides are used, preferred are bromides of lithium, sodium or potassium, or tetrabutylammonium bromide, in an amount that can range between about 0.1 to 1.0 equivalents, preferably about 0.2 equivalents. The preferred salt is lithium bromide.

Useful solvents are dimethylformamide (DMF) or, preferably, dimethylacetamide (DMA), with the optional addition of water in a volume comprised between about 0.1 to 1 with respect to the volume of the organic solvent.

By operating under the conditions described above, compound (IV) is obtained with high yield and with excellent regio- and stereoselectivity.

In the third step of the process of the invention, the benzyl protecting group in the benzyl ester group of compound (IV) is removed through hydrogenolysis, resulting in the corresponding acid (V). The reaction is carried out operating with hydrogen at atmospheric pressure in the presence of a hydrogenation catalyst. The catalyst may be Nickel Raney, palladium hydroxide, platinum on carbon, platinum oxide or, preferred, palladium on carbon. The reaction takes place in a solvent that may be chosen among methanol (preferred), ethanol, isopropanol, tetrahydrofuran (THF) or ethyl acetate (AcOEt). The resulting acid may be purified through crystallization, optionally having previously transformed it into a salt thereof.

In the fourth step of the process of the invention, the double bond conjugated to the phenyl ring in compound (V) is hydrogenated resulting in the correspondent saturated compound (VI).

This step can be carried out according to two alternative syntethic pathways.

According to a first possibility (reaction 4a in the general process), compound (V) (or a salt thereof) is hydrogenated under the same conditions described for the previous step. In this case, acid (VI) is obtained in the form of a racemic mixture, that must then be resolved via the formation of diastereoisomeric salts with chiral amines such (*R*)- or (*S*)-1-phenylethylamine, (-)-cinchonidine, or, preferred, (+)-cinchonine. Optical resolution takes place with a molar ratio racemic acid : chiral amine comprised between 1 : 0.5 and 1 : 1, preferably 1 : 0.5 in a protic or aprotic solvent such as an alcohol, an ester (e.g., ethyl acetate) or a ketone, such as methyl ethyl ketone (MEK) or, preferably, acetone. The enantiomeric eccess of the acid can be enhanced by re-crystallizing the salt with the chiral amine. At the end of the resolution, the optically active amine is easily recovered and the undesired isomer of the acid can be racemized and fed back to the optical resolution process.

According to the second possibility (reaction 4b in the general process), compound (V) (or a salt thereof) can be subjected to direct asymmetric hydrogenation, by using rhodium or ruthenium based precatalysts and chiral phosphines.

In case the fourth step of the process has been carried out on a salt of compound (V), a corresponding salt of compound (VI) is obtained, which has to be converted to the free acid before the next step.

The fifth step of the process of the invention consists in a Curtius reaction by which the free carboxylic acid group in compound (VI) is transformed into a protected amine in the form of a carbamate group.

This step requires a first reaction of activation of the carboxylic acid via its transformation into the corresponding acyl chloride or a mixed anhydride according to methods known in the field, followed by reaction with sodium or potassium azide to yield the corresponding acyl azide; the same transformation can be obtained more conveniently by direct reaction of the carboxylic acid with diphenylphosphoryl azide (DPPA) in the presence of an organic base such as a tertiary amine. Upon heating, the acyl azide releases a nitrogen molecule and rearranges to isocyanate; if the reaction is carried out in the presence of an alcohol, the final result is a carbamate of formula (VII), in which R is the alkyl group corresponding to the chosen alcohol. Alcohols useful for the aims of the present invention are 2,2,2-trichloroethanol, allyl alcohol, *tert*-butanol, preferably methanol, ethanol, isopropanol and more preferably benzyl alcohol.

The reaction can be carried out in an inert solvent such as THF or, preferably, toluene; in this case, the amount of alcohol employed may vary between 1 equivalent (based on the acid) and a volume ratio with the solvent of about 1:1. Alternatively, the reaction can be carried out in the alcohol as solvent, avoiding the use of the inert solvent.

In case of direct transformation of the carboxylic acid into the azide, the molar ratio DPPA : carboxylic acid may vary between about 1 : 1 and 1 : 2, preferably about 1:1. The tertiary amine can be ethyl di-isopropyl amine, *N-*methyl morpholine, tributylamine or, preferably, triethylamine.

The Curtius reaction takes place at a temperature comprised between about 50°C and the reflux temperature of the solvent, and preferably between about 75 and 110°C.

The sixth step of the process of the invention consists in the hydrolysis of the methyl ester of carbamate (VII) resulting into compound (VIII), according to methods known in the field; for example the reaction can be carried out with alkaline hydroxides such as sodium or potassium hydroxide in water or water-organic solvent mixtures, followed by acidification to obtain the free acid.

The seventh step of the process of the invention consists in the formation of the amide bond between carboxylic acid (VIII) and compound (IX), 3-(trifluoromethyl)-5,6,7,8-tetrahydro-[1,2,4]-triazolo-[4,3-a]-pyrazine, producing compound (X).

This reaction can be carried out according to several different ways.

A first possibility is to activate the carboxylic acid with carbonyldiimidazole and then adding the pyrazine (IX) to the reaction mixture. The reaction is carried out in an inert solvent such as acetonitrile (ACN), dimethylacetamide (DMA) or, preferably, THF. In the first phase (activation of the carboxylic acid) the temperature is mantained between about 0 and 25°C, while in the phase of reaction with the pyrazine the temperature is maintained between about 50°C and the reflux temperature of the solvent. It is also possible to use pyrazine (IX) hydrochloride by adding a suitable organic base to the reaction mixture, such as a tertiary amine, for example triethylamine.

Alternatively, the formation of the amide can be obtained by using condensing agents and additives known in the field, such as dicyclohexylcarbodiimide (DCC) or *N-*(3-dimethylaminopropyl)-N'-ethylcarbodiimide (EDC), with the optional addition of further additives such as *N-*hydroxybenzotriazole (*N-*HOBt)

The eighth step of the process of the invention is the removal of the carbamate group in compound (X) with deprotection of the amine, that yields Sitagliptin (I). This step is carried out according to methods known in the field, see e.g. Theodora W. Green, Protective Groups in Organic Synthesis, John Wiley & Sons (1999). For example, in the case of *tert*-butyl carbamate deprotection with an acid as trifluoroacetic acid is suitable; in the case of benzyl carbamate a hydrogenolysis as described for the third step of the synthesis is appropriate; and in the case of methyl, ethyl or isopropyl carbamate an alkaline hydrolysis is suitable although the yield of the product is lower due to the harsh reaction conditions.

Compounds (IV), (V), (VI), and compounds (VII), (VIII) and (X) in which R = benzyl, produced as intermediates during the process of the invention (and their salts in case of acid compounds) are new compounds, and constitute further objects of the invention.

The invention will be further described by the following examples.

### EXAMPLE 1

This example is about the synthesis of 4-Methoxy-2-methylene-4-oxobutanoic acid, intermediate in the production of compound (III) (first part of step 1 of the process of the invention).

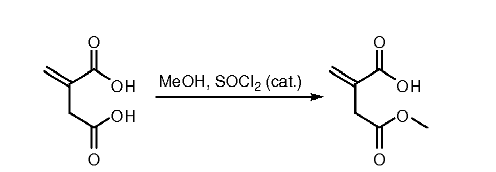

SOCl₂ (11.43, 0.096 mol) is slowly added at 0 °C to a solution of itaconic acid (250.0 g, 1.92 mol) in MeOH (1000 mL).

The mixture is then stirred under nitrogen at room temperature for 20 hours, monitoring by TLC (AcOEt-hexane 8:2, KMnO₄).

The reaction mixture is then poured into a solution of sodium carbonate (122 g, 1.15 mol) in water (750 mL), methanol is evaporated under reduced pressure and the aqueous phase is washed with isopropyl acetate (150 mL). The aqueous phase is diluted with water (150 mL) and the pH corrected to 2 by addition of concentrated sulphuric acid. The aqueous phase is extracted three times with isopropyl acetate (750, 300 and 150 mL) and the combined organic extracts are concentrated to a residue to obtain the product (233 g, 84% as a colourless solid).

The same product is obtained using Nafion NR₅₀, (0.05 eq) as the acidic catalyst (reaction time 60 hours at room temperature).

¹H NMR (400 MHz, CDCl₃) δ 6.50 (s, 1 H), 5.86 (d, *J*=0.7 Hz, 1 H), 3.73 (s, 3H), 3.37 (m, 2H).

### EXAMPLE 1B

This example is about the synthesis of 1-benzyl-4-methyl-2-methylenesuccinate, compound (III) (completion of step 1 of the process of the invention).

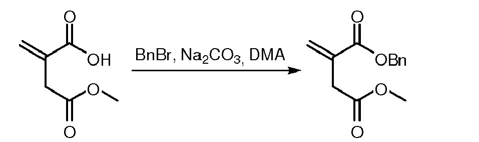

Na₂CO₃ (220 g, 2.081 mol) and benzyl bromide (90 mL, 0.763 mol) are added to a solution of 4-methoxy-2-methylene-4-oxobutanoic acid (100 g, 0.694 mol) in DMA (500 mL).

The mixture is stirred for 18 hours under nitrogen at room temperature monitoring by TLC (hexane-AcOEt 7:3, KMnO₄).

After complete consumption of the starting material, water (1000 mL) is added and the mixture is extracted with toluene (2 x 500 mL). The combined organic layers are washed with water (2 x 250 mL), and the solvent is removed under reduced pressure. The product is purified by distillation under reduced pressure (53 Pa, b.p. 140-150 °C) obtaining a colourless oil (136 g, 84%).

¹H NMR (400 MHz, CDCl₃) δ 7.42-7.32 (m, 5H), 6.41 (s, 1 H), 5.76 (s, 1 H), 5.23 (s, 2H), 3.67 (s, 3H), 3.39 (s, 2H).

### EXAMPLE 2

This example illustrates step 2 of the process of the invention: Heck coupling of 1-bromo-2,4,5-trifluorobenzene with 1-benzyl-4-methyl-2-methylenesuccinate with selective formation of (*E*)-1-benzyl-4-methyl-2-(2,4,5-trifluorobenzylidene)succinate, compound (IV).

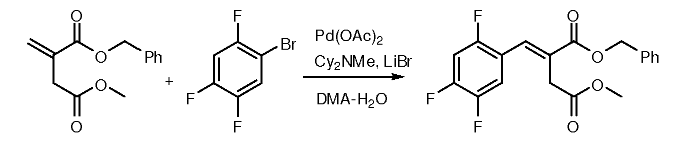

A 1000 mL round bottomed flask fitted with magnetic stirrer, thermometer, nitrogen inlet and reflux condenser is charged with 1-benzyl-4-methyl-2-methylenesuccinate (100.0 g, 0.43 mol), 1-bromo-2,4,5-trifluorobenzene (124.0 g, 0.59 mol), methyl dicyclohexyl amine (127.0 g, 0.65 mol), dimethyl acetamide (215 mL), water (53 mL) and lithium bromide (7.5 g, 0.09 mol). The mixture is degassed with three vacuum-nitrogen cycles; palladium acetate (260 mg, 1.16 10⁻³ mol) is then added as reaction catalyst.

The mixture is heated with stirring at 95-98 °C for 22 hours monitoring by GC until > 95% conversion, then cooled to room temperature, added of water (500 mL) and toluene (250 mL) and filtered on a Celite pad. The organic phase is washed with 1N HCl (200 mL), dried by azeotropic distillation under reduced pressure, treated with coarse silica gel (20 g), filtered and concentrated to a residue (128 g).

Gas-chromatographic analysis of the crude product shows the following composition: 87% (*E*)-1-benzyl-4-methyl-2-(2,4,5-trifluorobenzylidene)succinate, 7% (*Z*)-1-benzyl-4-methyl-2-(2,4,5-trifluorobenzylidene)succinate, 4% (*E*)- and (*Z*)-1-benzyl-4-methyl 2-(2,4,5-trifluorobenzyl)maleate, 2% starting material.

An analytical sample of pure (*E*)-1-benzyl-4-methyl-2-(2,4,5-trifluorobenzylidene)succinate is obtained by flash chromatography with gradient elution 0→20% AcOEt in hexane.

¹H NMR (300 MHz, CDCl₃) δ 7.77 (s, 1H), 7.40-7.34 (m, 5H), 7.22 (ddd, *J*=15.3, 8.6, 6.7 Hz, 1 H), 6.97 (ddd, *J*=15.9, 15.9, 6.4 Hz, 1 H), 5.26 (s, 2H), 3.66 (s, 3H), 3.43 (s, 2H).

### EXAMPLE 3

This example illustrates step 3 of the process of the invention, production of (*E*)-4-Methoxy-4-oxo-2-(2,4,5-trifluorobenzylidene)butanoic acid, compound (V). 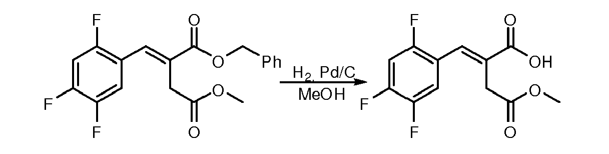 Crude (*E*)-1-benzyl-4-methyl-2-(2,4,5-trifluorobenzylidene)succinate (135 g) is dissolved in methanol (500 mL) and added of Pd/C (10% w/w, 50% water, 3.94 g) under nitrogen. Three cycles of vacuum-hydrogen (balloon) are performed and the mixture is stirred overnight at 20 °C. After filtration of the catalyst and removal of methanol the product is obtained as a colourless oil (133 g). HPLC analysis shows 6% of reduction of the C=C double bond. Crystallization from methyl *t-*butyl ether and hexane affords the product as a colourless solid, m.p. (DSC) 124.2 °C.

¹H NMR (300 MHz, CDCl₃) δ 11.0 (bs, 1 H), 7.87 (s, 1 H), 7.25 (ddd, *J*=15.3, 8.6, 6.7 Hz, 1 H), 6.98 (ddd, *J*=15.9, 15.9, 6.4 Hz, 1 H), 3.74 (s, 3H), 3.44 (s, 2H)

¹³C NMR (75 MHz, CDCl₃) δ 173.6, 172.8, 156.1 (dd, *J₁*=242.0, *J₂*=9.0 Hz), 151.2 (dt, *J₁*=254.0, *J₂=J₃*=14.0 Hz), 147.2 (ddd, *J₁*=214.0, *J₂*=14.0 *J₃*=4.0 Hz), 137.0, 130.4, 119.0 (dt, *J₂*=15.0, *J₃*=4.5 Hz), 118.1 (d, *J₂*=19.5 Hz), 106.5 (dd, *J₂*=27.5, *J₂*=21.2 Hz), 54.4, 35.3.

### EXAMPLE 4

Production of 4-Methoxy-4-oxo-2-(2,4,5-trifluorobenzyl)butanoic acid (first part of step 4a of the process of the invention).

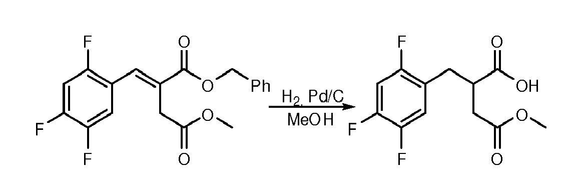

Pd/C (10% 50 mg) is added to (*E*)-1-benzyl-4-methyl-2-(2,4,5-trifluorobenzylidene)succinate (500 mg, 1.37 mmol) in MeOH (15 mL) and the mixture is hydrogenated at 3 bar for 12 hours at room temperature monitoring by TLC (AcOEt-hexane 8:2) or by HPLC.

The product (359 mg, 95%) is isolated by concentration of the reaction mixture after filtration on a Celite pad. Colourless solid, m.p. (DSC) 83.5 °C

¹H NMR (400 MHz, CDCl₃) δ 7.09-7.01 (m, 1 H), 6.98-6.90 (m, 1 H), 3.70 (s, 3H), 3.23-3.14 (m, 1 H), 3.07 (dd, *J*=6.7, 14.0Hz, 1 H), 2.88 (dd, *J*=7.7, 13.9Hz, 1 H) 2.71 (dd, *J*=8.5, 17.0Hz, 1 H) 2.50 (dd, *J*=5.2, 17.0Hz, 1 H).

¹³C NMR (100 MHz, CDCl₃) δ 179.3, 171.7, 156.6 (ddd, *J₁*=245.0 *J₂*=9.0, *J₃*=3.0 Hz), 150.9, 145.7, 121.0 (ddd, *J₁*=18.0, *J₂*=5.0, *J₃*=4.0Hz), 119.2 (ddd, *J₁*=19.1, *J₂*=5.9, *J₃*=1.3 Hz), 106.0 (ddd, *J₁*=28.4, *J₂*=20.8, *J₃*=0.5 Hz), 51.96, 41.39, 34.53, 29.94

### EXAMPLE 4A

This example is about the obtainment of (*R*)-4-Methoxy-4-oxo-2-(2,4,5-trifluorobenzyl)butanoic acid (compound VI) by resolution of the racemic acid with (*S*)-1-phenylethylamine (second part of step 4a of the process of the invention).

(*S*)-1-phenylethylamine (2.2 g, 18.1 mmol) is added to a solution of racemic 4-methoxy-4-oxo-2-(2,4,5-trifluorobenzyl)butanoic acid (5.0 g, 18.1 mmol) in acetonitrile (25 mL). The mixture is heated to reflux, then is cooled to 35 °C (onset of crystallization). The mitxture is then slowly cooled to 25 °C, aged for 30 minutes at the same temperature, filtered and the solid is washed with cold acetonitrile. Yield 3.5 g of free optically enriched acid.

Chiral HPLC shows an enantiomeric ratio of 35 : 65.

HPLC conditions: Chiracel AD 4.6 x 250 mm, Mobile phase: hexane - *i-*PrOH-EtOH - trifluoroacetic acid (TFA) 75:14:11:0.3, flow 1 ml/min.
(-)-(*S*) acid Rₜ = 11.12 min;
(+)-(*R*) acid Rₜ = 12.36 min

### EXAMPLE 4B

This example is about the obtainment of (*R*)-4-Methoxy-4-oxo-2-(2,4,5-trifluorobenzyl)butanoic acid (compound VI) by resolution of the racemic acid with (+)-cinchonine (second part of step 4a of the process of the invention).

(+)-Cinchonine (2.66 g, 9.0 mmol) is added to a solution of racemic 4-methoxy-4-oxo-2-(2,4,5-trifluorobenzyl)butanoic acid (5.0 g, 18.1 mmol) in acetone (60 mL). The mixture is heated to reflux, then is cooled to 25 °C, then slowly cooled to 0 °C, aged for 60 minutes at the same temperature, filtered, and the resulting solid is washed with cold acetone. Yield 2.5 g of free optically enriched acid.

Chiral HPLC shows an enantiomeric ratio of 20 : 80.

The cinchonine salt is recristallyzed from acetone to obtain an enantiomeric ratio of 3 : 97.

### EXAMPLE 4C

This example is about the obtainment of (*S*)-4-Methoxy-4-oxo-2-(2,4,5-trifluorobenzyl)butanoic acid by resolution of the racemic acid with (-)-cinchonidine.
(-)-Cinchonidine (5.33 g, 18.1 mmol) is added to a solution of 4-methoxy-4-oxo-2-(2,4,5-trifluorobenzyl)butanoic acid (5.0 g, 18.1 mmol) in acetone (30 mL). The mixture is heated to reflux, then is cooled to 25 °C, then slowly cooled to 0 °C, aged for 60 minutes at the same temperature, filtered, and the solid is washed with cold acetone. Yield 2.5 g of free optically enriched acid.
Chiral HPLC shows an enantiomeric ratio of 77 : 23.

### EXAMPLE 4D

Example 4C is repeated in the same conditions, but using 2.66 g (9.0 mmol) of (-)-cinchonidine (one half of the amount used in Example 4C)
The yield is 2.2 g of free optically enriched acid.
Chiral HPLC shows an enantiomeric ratio of 92 : 8.

### EXAMPLE 5

This example illustrates step 5 of the process of the invention, production of (*R*)-Methyl-3-(tert-butoxycarbonylamino)-4-(2,4,5-trifluorophenyl)butanoate, compound (VII) with R = tert-butyl.

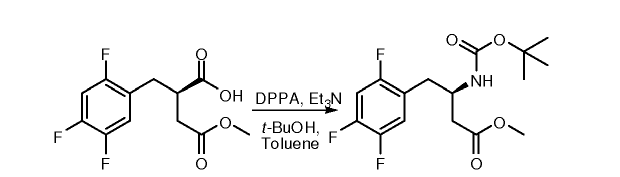

Triethylamine (400 µL, 2.7 mmol) is added to a solution of the (*R*)-acid (500 mg, 1.8 mmol) in toluene (10 mL) and *t-*butanol (0.2 mL). The mixture is heated to 80°C under nitrogen. A solution of diphenylphosphoryl azide (500 mg, 1.8mmol) in toluene (1.50 mL) is added *via* a syringe pump in 30 minutes. The mixture is then heated to reflux for 5 hours, monitoring by TLC (hexane-AcOEt 8 :2) then cooled to room temperature and water (5 mL) is added. The organic phase is diluted with toluene (10 mL) and washed twice with brine and concentrated to a residue which is purified by flash chromatography with gradient elution 0→50% AcOEt in hexane, collecting the title product (434 mg, 69%) as a colourless solid.

¹H NMR (400 MHz, CDCl₃) δ 7.11-7.01 (m, 1 H), 6.96-6.86 (m, 1 H), 5.14 (d, *J*=8.3Hz, 1 H), 4.20-4.09 (m, 1 H), 3.73 (s, 3H), 2.93-2.80 (m, 2H), 2.64-2.49 (m, 2H) 1.40 (s, 9H).

¹³C NMR (100 MHz, CDCl₃) δ 171.7, 157.9 (ddd, J₁=247.0, J₂=12.0, J₃=3.0 Hz), 154.9, 148.9 (ddd, J₁=250.0z, J₂=26.0, J₃=13.0 Hz), 146.2 (ddd, J₁=244.0, J₂=13.0 Hz, J₃=3.0 Hz), 121.2 (ddd, J₁=19.0, J₂=9.0, J₃=5.0), 118.9 (dd, J₁=18.9, J₂=5.6), 105.2 (dd, J₁=28.0 Hz, J₂=20.0 Hz), 79.5, 51.7, 47.6, 37.8, 32.90, 28.1

### EXAMPLE 5A

This example illustrates step 5 of the process of the invention, production of (*R*)-Methyl-3-(isopropoxycarbonylamino)-4-(2,4,5-trifluorophenyl)butanoate, compound (VII) with R = isopropyl.

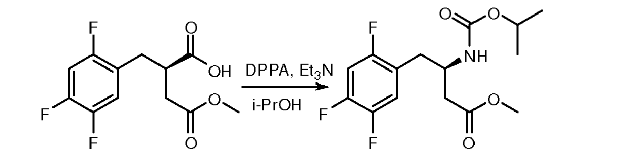

The (*R*)-acid (5.53 g, 20.4 mmol) is dissolved in isopropanol (55 mL), added of triethylamine (2.98 mL, 21.4 mmol) and the mixture is heated at reflux under nitrogen. Diphenyl phosphoryl azide (5.51 g, 20.4 mmol) is added dropwise and the mixture is stirred two hours monitoring by TLC (hexane-AcOEt 8:2) or HPLC until complete consumption of the acid.

The mixture is added of water (110 mL) and the mixture is cooled to room temperature with stirring. The precipitated product is filtered and washed with water.

The title product (6.0 g, 89% yield) is obtained as a colourless solid with 98.5% purity by HPLC at 210 nm.

¹H NMR (400 MHz, CDCl₃) δ 7.03 (ddd, *J*=15.3, 8.6, 6.7 Hz, 1H), 6.89 (ddd, *J*=15.9, 15.9, 6.4 Hz, 1 H), 5.19 (d, 1 H, NH), 4.81 (sept, *J*=6.4 Hz, 1 H), 4.22-4.10 (m, 1 H), 3.70 (s, 3H), 2.86 (d, *J*=7.0 Hz, 2H), 2.62-2.48 (m, 2H), 1.25-1.05 (m, 6H)

### EXAMPLE 5B

This example illustrates step 5 of the process of the invention, production of (*R*)-Methyl-3-(benzyloxycarbonylamino)-4-(2,4,5-trifluorophenyl)butanoate, compound (VII) with R = benzyl.

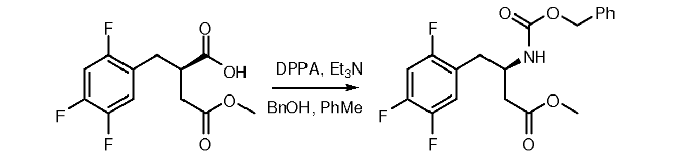

The (*R*)-acid (1.00 g, 3.62 mmol) is dissolved in toluene (10 mL), added of triethylamine (530 µL, 3.80 mmol) and the mixture is heated to 80 °C. Diphenyl phosphoryl azide (1.0 g, 3.62 mmol) is added dropwise followed by benzyl alcohol (398 mg, 3.62 mmol).

The reaction mixture is stirred at 80 °C for two hours monitoring by TLC (hexane-AcOEt 8:2) or HPLC until complete consumption of the acid.

Water (20 mL) is then added and the mixture is cooled to room temperature, the phases are separated and the organic phase is concentrated at atmospheric pressure. The residue (yellow solid) is dissolved in methanol (20 mL) and added of water (10 mL). The crystallized product is filtered and washed with water, obtaining the title compound as a colourless solid (1.09 g, 79% yield).

¹H NMR (300 MHz, CDCl₃) δ 7.37-7.27 (m, 5H), 7.05-6.97 (m, 1H), 6.90-6.82 (m, 1 H), 5.37 (d, *J*=9.2 Hz, NH), 5.05 & 5.00 (AB system, *J*=12.3 Hz, 2 x 1 H), 4.25-4.12 (m, 1 H), 3.68 (s, 3H), 2.90-2.85 (m, 2H), 2.63-2.48 (m, 2H)

### EXAMPLE 6

This example illustrates step 6 of the process of the invention, hydrolisys of the methyl-ester group of the carbamate obtained in example 5, producing (*R*)-3-(*Tert-*butoxycarbonylamino)-4-(2,4,5-trifluorophenyl)butanoic acid, compound (VIII) with R = tert-butyl.

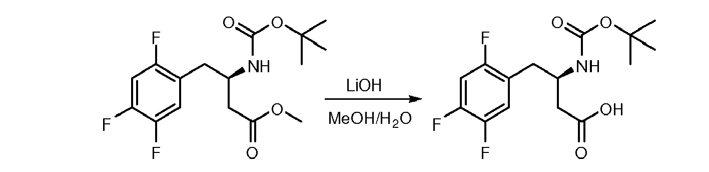

Lithium hydroxide (144 mg, 6.0 mmol) and water (3.0 mL) are added to a solution obtained by dissolving 400 mg (1.15 mmol) of (*R*)-methyl-3-(tert-butoxycarbonylamino)-4-(2,4,5-trifluorophenyl)butanoate in MeOH (10 mL).

The mixture is stirred at room temperature for 24 hours monitornig by TLC (hexane-AcOEt 8 : 2)

When the ester is completely hydrolized, methanol is removed under reduced pressure and the residue is taken up in AcOEt (20 mL) and 1 N HCl is added (5 mL). The phases are separated and the organic layer is washed with brine (5 mL), dried over sodium sulfate and concentrated to a residue, affording the title product as a pale yellow solid (364 mg, 95%)

¹H NMR (400 MHz, CDCl₃) δ: 7.13-7.03 (m, 1 H), 6.98-6.88 (m, 1 H), 5.09 (d, *J*=8.3Hz, 1 H), 4.25-4.04 (m, 1 H), 2.98-2.83 (m, 2H), 2.72-2.45 (m, 2H), 1.40 (s, 9H).

### EXAMPLE 6A

This example illustrates step 6 of the process of the invention, hydroysis of the methyl-ester group of the carbamate obtained in example 5A, producing (*R*)-3-(isopropoxycarbonylamino)-4-(2,4,5-trifluorophenyl)butanoic acid, compound (VIII) with R = isopropyl.

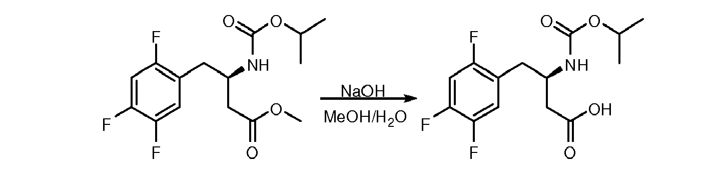

Sodium hydroxide (1.06 g, 26.5 mmol) and water (5.0 mL) are added to a solution of (*R*)-methyl 3-(isopropoxycarbonylamino)-4-(2,4,5-trifluorophenyl)butanoate (5.90 g, 17.7 mmol) in MeOH (50 mL).

The mixture is stirred at room temperature for 4 hours monitornig by HPLC.

When the ester is completely hydrolyzed, water (100 mL) is added, the mixture is cooled to room temperature and the pH is adjusted to 2 by addition of 1 N HCl. The precipitated solid is filtered and washed with water and dried under vacuum at 60 °C to obtain the title compound in quantitative yield.

¹H NMR (300 MHz, d₆-dmso) δ 12.3 (bs, 1 H), 7.52-7.38 (m, 1 H), 7.35-7.19 (m, 1 H), 6.97 & 6.65 (2 x d, J=9.2 Hz, 0.9 & 0.1 H, NH), 4.57 (sept, J=6.1 Hz, 1 H), 4.10-3.93 (m, 1 H), 2.84 & 2.56 (dd, J=12.8, 9.2 Hz, 2 x 1 H), 2.44-2.33 (m, 2H), 1.09 & 0.99 (2 x d, J=6.4 Hz, 2 x 3H)

### EXAMPLE 6B

This example illustrates step 6 of the process of the invention, hydrolysis of the methyl-ester group of the carbamate obtained in example 5B, producing (*R*)-3-(benzyloxycarbonylamino)-4-(2,4,5-trifluorophenyl)butanoic acid, compound (VIII) with R = benzyl.

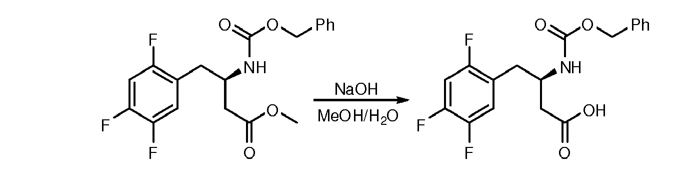

The hydrolysis is carried out as described in Example 6A. The crude acid is crystallized in 87% yield from methanol-water.

¹H NMR (300 MHz, d₆-dmso) δ 7.38-7.24 (m, 5H), 7.07-6.97 (m, 1H), 6.91-6.82 (m, 1 H), 6.0 & 5.34 (2 x bs, 1 H overall, 2 NH rotamers), 5.06 & 5.01 (AB system, J=11.6 Hz, 2 x 1 H), 4.3-4.1 (m, 1 H), 2.92-2.80 (m, 2H), 2.70-2.50 (m, 2H)

### EXAMPLE 7

This example is about step 7 of the process of the invention, production of *tert-*butyl-4-oxo-4-(3-(trifluoromethyl)-5,6-dihydro-[1,2,4]triazolo[4,3-a]pyrazin-7(8*H*)-yl)-1-(2,4,5-trifluorophenyl)butan-2-yl-carbamate, compound (X) with R = tert-butyl (amide formation with EDC/*N-*HOBT)

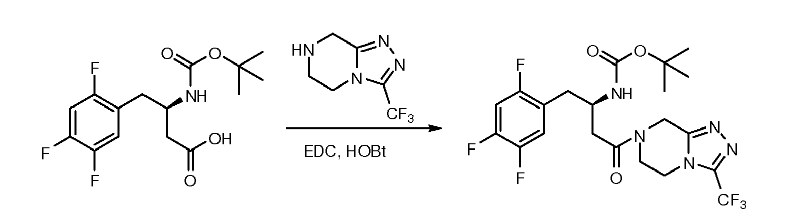

*N-*hydroxybenzotriazole (150 mg, 1.08mmol) is added at 0 °C to a solution of (*R*)-3-(tert-butoxycarbonylamino)-4-(2,4,5-trifluorophenyl)butanoic acid (300 mg, 0.90 mmol) and 3-(trifluoromethyl)-5,6,7,8-tetrahydro-[1,2,4]triazolo[4,3-a]pyrazine (170 mg, 0.90 mmol) in CH₂Cl₂ (10ml); after 10 minutes EDC (260 mg, 1.35mmol) is added.

The mixture is stirred under nitrogen at room temperature for 4 hours monitoring by TLC (AcOEt).

The reaction is quenched by addition of a saturated solution of NaHCO₃ (5 mL); after phase separation the organic layer is washed with brine (5 mL), dried over sodium sulphate, concentrated to a residue and purified by flash chromatography eluting with AcOEt obtaining the title product (406 mg, 86%) as a off-white solid.

¹H NMR (400 MHz, CDCl₃) δ 7.14-7.04 (m, 1 H), 6.97-6.83 (m, 1 H), 5.32-5.24 (bs, 1 H), 5.16-5.00 (m, 1 H), 4.94 (s, 1 H), 4.33-3.94 (m, 5H), 3.02-2.62 (m, 4H) 1.38 (s, 9H).

### EXAMPLE 7A

Example 7 is repeated with a different carbamate as starting compound, producing (*R*)-Isopropyl-4-oxo-4-(3-(trifluoromethyl)-5,6-dihydro-[1,2,4]triazolo[4,3-a]pyrazin-7(8*H*)-yl)-1-(2,4,5-trifluorophenyl)butan-2-yl-carbamate, compound (X) with R = isopropyl (amide formation with carbonyldiimidazole and triazolopyrazine free base)

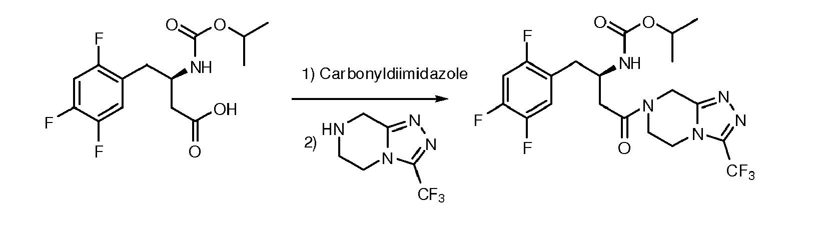

Carbonyldiimidazole (632 mg, 3.9 mmol) is added in one portion at 0 °C under nitrogen to a solution of (*R*)-3-(isopropoxycarbonylamino)-4-(2,4,5-trifluorophenyl)butanoic acid (1.04 g, 3.25 mmol) in dry THF (10 mL). When CO₂ evolution ceases, a solution of 3-(trifluoromethyl)-5,6,7,8-tetrahydro-[1,2,4]triazolo[4,3-a]pyrazine (625 mg, 3.25 mmol) in THF (3 mL) is added and the mixture is heated to reflux for 12 hours; the pyazine has been obtained apart from the corresponding hydrochloride by neutralization with 1N NaOH in water and concentration to a residue, followed by dissolution in toluene and filtration of NaCl.

After complete reaction by HPLC, THF is removed under reduced pressure and the residue taken up in toluene (50 mL), washed with water and brine and concentrated to a residue that is crystallized from methanol - water 2:1 to afford the title product as a colourless solid (1.46 g, 91 %).

### EXAMPLE 7B

Example 7 is repeated with a different carbamate as starting compound, producing (*R*)-Benzyl-4-oxo-4-(3-(trifluoromethyl)-5,6-dihydro-[1,2,4]triazolo[4,3-a]pyrazin-7(8*H*)-yl)-1-(2,4,5-trifluorophenyl)butan-2-yl-carbamate, compound (X) with R = benzyl (amide formation with carbonyl diimidazole and triazolopyrazine hydrochloride-triethylamine)

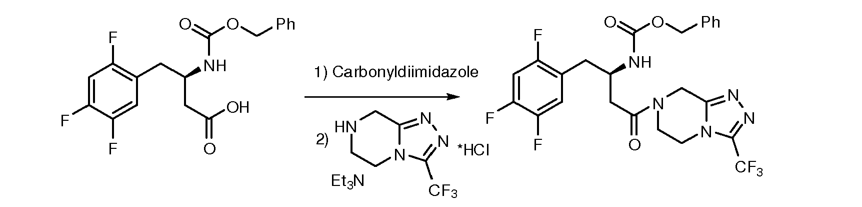

Carbonyl diimidazole (510 mg, 3.15 mmol) is added in one portion at 0 °C under nitrogen to a solution of (*R*)-3-(benzyloxycarbonylamino)-4-(2,4,5-trifluorophenyl)butanoic acid (1.00 g, 2.62 mmol) in dry THF (5.0 mL). After 10 minutes a white precipitate appears, triethylamine (369 µL, 2.62 mmol) is added followed by 3-(trifluoromethyl)-5,6,7,8-tetrahydro-[1,2,4]triazolo[4,3-a]pyrazine hydrochloride (599 mg, 2.62 mmol) and the mixture is heated to reflux overnight. The mixture is then cooled to room temperature and water (10 mL) and toluene (10 mL) are added, the phases are separated and the organic phase is washed with water (3 x 5 mL), dried over sodium sulphate and concentrated under reduced pressure to a residue, pure enough by HPLC to carry out the debenzylation step.

¹H NMR (300 MHz, d₆-dmso, 100 °C) δ 7.4-7.2 (m, 7H), 6.90 (bs, 1H) 4.97 & 4.90 (AB system, J= 12.9 Hz, 2 x 1 H), 4.92 (s, 2H) 4.26-4.14 (m, 3H), 3.97 (t, J=5.5 Hz, 2H), 2.92 & 2.67 (2 x dd, J=14.1, 4.9 Hz, 2 x 1 H), 2.83-2.72 (m, 2H)

### EXAMPLE 8

This example refers to step 8 of the process of the invention, production of Sitagliptin base (compound (I)).

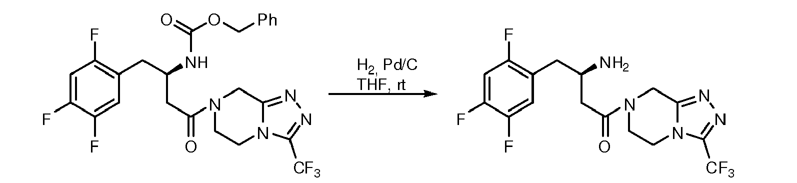

Pd/C (10%, 50% water wet, 256 mg, 0.12 mmol, 5% mol) is added to a solution of (*R*)-benzyl-4-oxo-4-(3-(trifluoromethyl)-5,6-dihydro-[1,2,4]triazolo[4,3-a]pyrazin-7(8*H*)-yl)-1-(2,4,5-trifluorophenyl)butan-2-ylcarbamate (1.3 g, 2.40 mmol) in THF (5.0 mL) and the mixture is hydrogenated at atmospheric pressure overnight at room temperature. The catalyst is filtered and the solution is concentrated to a residue, obtaining Sitagliptin as a white foam in quantitative yield.

## Claims

1. Process for the production of Sitagliptin of formula (I) 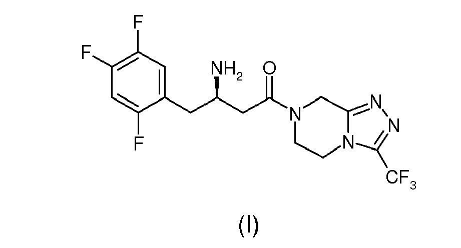 comprising the steps of:
1) reacting itaconic acid (II) first with methyl alcohol and then with benzyl bromide, obtaining 1-benzyl-4-methyl-2-methylenesuccinate (III): 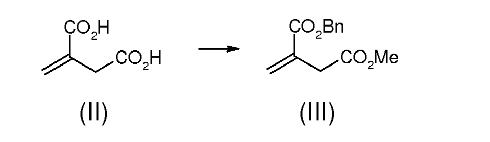 in which Bn indicates the benzyl radical;
2) reacting compound (III) with 1-bromo-2,4,5-trifluorobenzene to obtain (*E*)-1-benzyl-4-methyl-2-(2,4,5-trifluorobenzylidene)succinate, compound (IV): 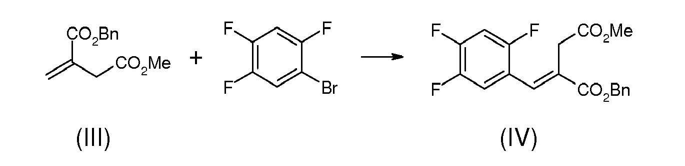
3) debenzylating compound (IV) to obtain (*E*)-4-methoxy-4-oxo-2-(2,4,5-trifluorobenzylidene)butanoic acid, compound (V): 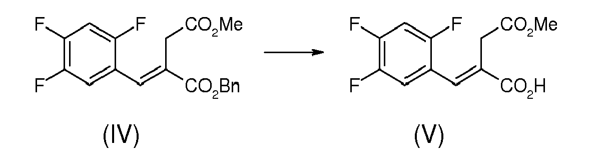
4) transforming compound (V) into (*R*)-4-Methoxy-4-oxo-2-(2,4,5-trifluorobenzyl)butanoic, compound (VI): 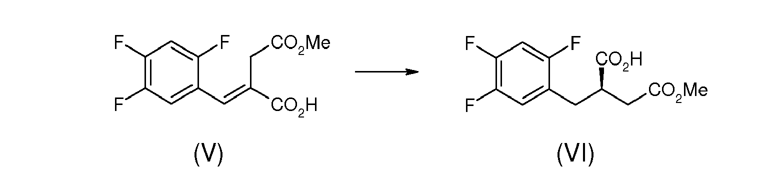 through 4a) catalytic hydrogenation followed by optical resolution, or 4b) asymmetric hydrogenation;
5) transforming compound (VI) through the Curtius reaction into a carbamate of general formula (VII): 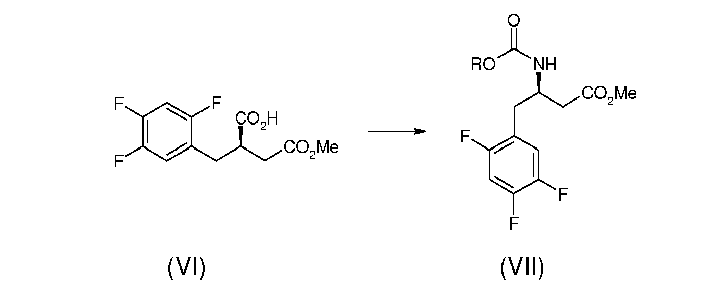
6) hydrolyzing the methyl-ester group of carbamate (VII) to obtain the corresponding acid (VIII): 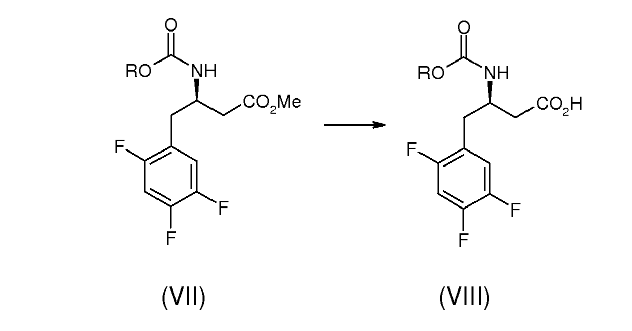
7) reacting acid (VIII) with 3-(trifluoromethyl)-5,6,7,8-tetrahydro-[1,2,4]triazolo[4,3-a]pyrazine, compound (IX), to obtain amide (X): 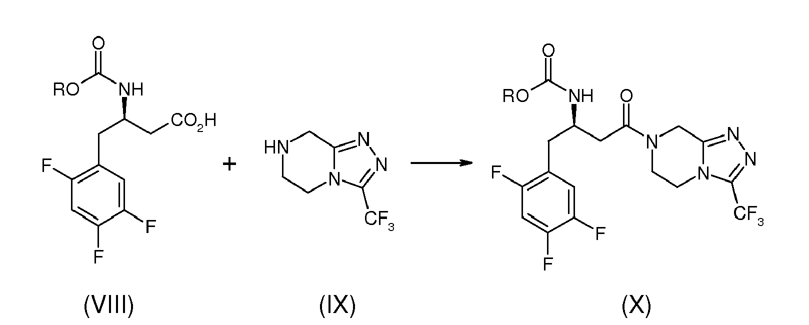
8) transforming the carbamate group of compound (X) into the corresponding amino group, thus obtaining Sitagliptin (I):

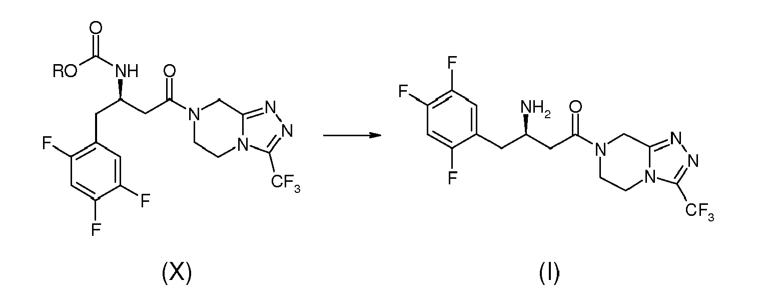

2. Process according to claim 1, wherein in step 2) compound (III) is reacted with 1-bromo-2,4,5-trifluorobenzene in the presence of a base, in an organic polar aprotic solvent, at a temperature comprised between about 50 and 150 °C, and in the presence of a palladium-based catalyst and optionally in the presence of an alkaline bromide or a tetraalkylammonium bromide.

3. Process according to claim 2, wherein said base is chosen among Na₂CO₃ or K₂CO₃, or tertiary amines, and its amount is comprised between about 1 and 2 equivalents; the solvent is chosen among dimethylformamide or, dimethylacetamide with the optional addition of water in a volume comprised between about 0.1 to 1 with respect to the volume of the organic solvent; and the catalyst is chosen among palladium chloride, palladium acetate or metallic palladium on carbon.

4. Process according to any of the preceding claims, wherein step 3) is carried out operating with hydrogen in the presence of a hydrogenation catalyst chosen among Nickel Raney, palladium hydroxide, platinum on carbon, platinum oxide or palladium on carbon, in a solvent chosen among methanol, ethanol, isopropanol, tetrahydrofuran or ethyl acetate.

5. Process according to any of the preceding claims, wherein step 4) is carried out operating with hydrogen in the presence of a hydrogenation catalyst chosen among Nickel Raney, palladium hydroxide, platinum on carbon, platinum oxide or palladium on carbon, in a solvent chosen among methanol, ethanol, isopropanol, tetrahydrofuran or ethyl acetate, and the product of hydrogenation is a racemic mixture that is then resolved via the formation of diastereoisomer salts with a chiral amine, with a molar ratio racemic acid : chiral amine comprised between 1 : 0.5 and 1 : 1, and in solvent chosen among an alcohol, an ester or a ketone.

6. Process according to claim 5, wherein said chiral amine is chosen among (*R*)-or (*S*)-1-phenylethylamine, (-)-cinchonidine, or (+)-cinchonine.

7. Process according to any of claims 1 to 4, wherein step 4) is carried out by subjecting compound (V) or a salt thereof to direct asymmetric hydrogenation by using rhodium or ruthenium based precatalysts and chiral phosphines.

8. Process according to any of the preceding claims, wherein step 5) acid (VI) is transformed via a Curtius reaction into of general formula carbamate (VII), in which R is chosen among methyl, ethyl, 2,2,2-trichloroethyl, allyl, iso-propyl, *tert*-butyl and benzyl, said reaction taking place at a temperature comprised between about 50 °C and the reflux temperature of the solvent and in an inert solvent chosen among THF, toluene, or an alcohol R-OH, in which R has the meaning above.

9. Process according to any of the preceding claims, wherein in step 7) acid (VIII) is activated with carbonyldiimidazole at a temperature between about 0 and 25 °C, followed by addition of pyrazine (IX) to the reaction mixture in an inert solvent chosen among acetonitrile, dimethylacetamide or THF at a temperature between about 50 °C and the reflux temperature of the solvent.

10. Process according to any of claims 1 to 8, wherein step 7) is carried out by adding to the reactants a condensing agent and an additive.

11. (*E*)-1-Benzyl-4-methyl-2-(2,4,5-trifluorobenzylidene)succinate of formula (IV).

12. (*E*)-4-Methoxy-4-oxo-2-(2,4,5-trifluorobenzylidene)butanoic acid of formula (V) and salts thereof.

13. 4-Methoxy-4-oxo-2-(2,4,5-trifluorobenzyl)butanoic acid in racemic form, in its enantiomeric (S)-form or in its enantiomeric (R)-form of formula (VI), and salts thereof.

14. (*R*)-Methyl-3-(benzyloxycarbonylamino)-4-(2,4,5-trifluorophenyl)butanoate, compound of formula (VII) in which R = benzyl.

15. (*R*)-3-(Benzyloxycarbonylamino)-4-(2,4,5-trifluorophenyl)butanoic acid, compound of formula (VIII) in which R = benzyl, and salts thereof.

16. (*R*)-Benzyl-4-oxo-4-(3-(trifluoromethyl)-5,6-dihydro-[1,2,4]triazolo[4,3-a]pyrazin-7(8*H*)-yl)-1-(2,4,5-trifluorophenyl)butan-2-yl-carbamate, compound of formula (X) in which R = benzyl.
